# EUROPEAN PATENT SPECIFICATION

(11) **EP 0 292 727 B1**
(45) Date of publication and mention of the grant of the patent: **20.10.1993**
(21) Application number: 88106852.2
(22) Date of filing: 28.04.1988
(51) Int. Cl.: C12Q 1/30, C12N 11/14, G01N 21/76, C12Q 1/28, C12N 11/10

(54) **Reagent for use in the measurement of hydrogen peroxide in sample, method of preparation of same as well as process and apparatus for use thereof in the measurement of hydrogen peroxide**
Reagens zur Verwendung bei der Messung von Wasserstoffperoxid in einer Probe, Verfahren für seine Herstellung sowie Verfahren und Vorrichtung für seinen Gebrauch bei der Messung von Wasserstoffperoxid
Réactif à employer pour la mesure de peroxyde d'hydrogène dans les échantillons, son procédé de préparation ainsi que le procédé d utilisation dans la mesure de peroxyde d hydrogène

(30) Priority: 28.04.1987 JP 103209/87; 28.04.1987 JP 103210/87
(43) Date of publication of application: 30.11.1988
(73) Proprietor: MEIDENSHA KABUSHIKI KAISHA, Shinagawa-ku Tokyo 141 (JP)
(72) Inventor: Kusumi, Miyoko, Koganei-shi Tokyo (JP); Matsuyuki, Akira, Sumida-ku Tokyo (JP); Fujie, Shinichi, Sakado-shi Saitama-ken (JP)
(74) Representative: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) References cited:
- EP-A- 0 178 790
- EP-A- 0 183 207
- EP-A- 0 223 479
- US-A- 3 677 903
- CHEMICAL ABSTRACTS, vol. 99, no. 5, 1st August 1983, page 225, no. 34881x, Columbus, Ohio, US; W.D. GEOGHEGAN et al.: "Adaptation of the Ngo-Lenhoff peroxidase assay for solid phase ELISA" & J. IMMUNOL. METHODS 1983, 60(1-2), 61-8

## Description

### BACKGROUND OF THE INVENTION

### (Field of The Invention)

The present invention relates to a reagent for use in the measurement of hydrogen peroxide in a sample, a method for preparing the same and a process for measuring the hydrogen peroxide in the sample by using the reagent. More specifically, the invention relates to a reagent which can be also used for the measurment of a small amount of hydrogen peroxide in a sample.

### (Description of The prior Art)

In clinical examinations, the content of hydrogen peroxide is often measured. When the quantitative analysis of a component, such as a glucose, a cholesterol, an amino acid, a polyamine or the like, in an organism is performed, the component is caused to react with an oxidase to produce hydrogen peroxide, add thereafter the hydrogen peroxide content is determined.

In the Enzyme Immnoassay (EIA) Method, an antigen or an antibody, such as a hormone, a protein or the like, are labelled with an oxidase, such as glucose oxidase or the like, and thereafter the latter is caused to react with a substrate, which is glucose when the oxidase is glucose oxidase, to produce hydrogen peroxide. The absorbance of the hydrogen peroxide obtained is thereafter measured by using a dye, such as 4-aminoantipyrine so that the content thereof can be determined, and thereby the antigen or the antibody can be analyzed.

Instead of the dye, chemiluminescent material, such as a luminol, a lucigenin and so forth, which reacts with a hydrogen peroxide to produce a light in the presence of a catalyst, can be used for the determination of the hydrogen peroxide. These methods are effective for the analysis of chemical components of the organism since the hydrogen peroxide content can be precisely measured.

In cases where the components of an organism are analyzed by the aforementioned methods, it is very important to accurately determine the content of hydrogen peroxide in a sample.

When the content of hydrogen peroxide is measured according to aforementioned methods, the luminescent reagent and the catalyst are mixed with various concentrations of hydrogen peroxide, the concentrations of which are previously determined, before the sample is measured. Thereafter, the quantities of emitted light due to the reactions between the luminescent reagent and the hydrogen peroxide in the presence of the catalyst are measured. On the basis of these results, a calibration curve representing the relationship between the hydrogen peroxide and the quantity of emitted light is made. Thereafter, the content of the hydrogen peroxide produced by the reaction between the sample and the oxidase is determined on the basis of the calibration curve.

However, when the content of the hydrogen peroxide is determined by causing the luminescent material to produce light in the presence of the catalyst according to the aforementioned method, it is difficult to accurately determine the content of the hydrogen peroxide in the sample if the content thereof is low, i.e. less than 10⁻⁶ mol/l. Because a small amount of hydrogen peroxide is included in the luminescent material, so that the proportion of the hydrogen peroxide in water solutions containing the luminescent material and the catalyst to that in the sample is relatively high when the content of the hydrogen peroxide in the sample is low.

Accordingly, it is a principal object of the present invention to provide an improved reagent for use in the measurement of hydrogen peroxide in a sample.

It is a particular object of the invention to provide an improved reagent by which low concentrations of hydrogen peroxide in a sample can be more accurately measured than by a conventional reagent.

It is another object of the invention to provide a method for the preparation of a reagent used in the measurement of hydrogen peroxide.

It is another object of the invention to provide a process for measuring the content of hydrogen peroxide by using a reagent.

It is a further object of the invention to provide an apparatus for measuring the quantity of light produced by the reaction between a sample and a reagent in the presence of a catalyst.

Further objects and advantages will appear hereinbelow.

### SUMMARY OF THE INVENTION

In accordance with the present invention the foregoing objects and advantages are readily obtained.

According to one aspect of the invention, a process for measuring the content of hydrogen peroxide in a sample comprises:
mixing a luminol reagent including a hydrogen peroxide with a catalase to decompose the hydrogen peroxide of the luminol reagent;
adding an inhibitor, by which the reaction between the catalase and the hydrogen peroxide is stopped, to the mixture of the catalase and the luminol reagent;
adding the luminol reagent to the sample; and
measuring the content of hydrogen peroxide in the sample.

In this case, the inhibitor is preferably a sodium azide.

According to another aspect of the invention, a process for measuring the content of hydrogen peroxide in a sample comprises:
mixing a luminol reagent including a hydrogen peroxide with a catalase, the concentration of which is 10 to 200 U/ml based on the total volume, to decompose the hydrogen peroxide of the luminol reagent;
adding the luminol reagent to the sample; and
measuring the content of hydrogen peroxide in the sample.

According to another aspect of the invention, a process for measuring the content of hydrogen peroxide in a sample comprising:
bringing a luminol reagent including a hydrogen peroxide into contact with an immobilized catalase to decompose the hydrogen peroxide in the luminol reagent;
adding the luminol reagent to the sample; and
measuring the content of hydrogen peroxide in the sample.

The reagent may be caused to pass through a column, in which the immobilized catalase is packed, to come into contact with the immobilized catalase. The immobilized catalase may comprise a catalase which is immobilized on a carrier formed by a reaction between a water insoluble carrier and a polyfunctional reagent.

According to another aspect of the invention, a reagent for use in the measurement of a hydrogen peroxide in a sample comprises a luminol reagent and a catalase, the concentration of which is 10 to 200 U/ml based on the total volume.

According to another aspect of the invention, a reagent for use in the measurement of hydrogen peroxide in a sample comprises a luminol reagent and an immobilized catalase.

The immobilized catalase may comprise a catalase immobilized on a carrier which is formed by a reaction between a water insoluble carrier and a polyfunctional reagent. The water insoluble carrier is preferably an aminopropyl-CPG (controlled pore glass). The polyfunctional reagent is prefarably a glutaraldehyde.

According to another aspect of the invention, a method for producing a reagent for used for use in the measurement of a hydrogen peroxide in a sample comprises the steps of:
providing a luminol reagent including hydrogen peroxide; and
adding a catalase to the luminol reagent to decompose the hydrogen peroxide therein, and thereafter removing said catalase remaining in the luminol reagent.

According to another aspect of the invention, a method for producing a reagent for use in the measurement of a hydrogen peroxide in a sample comprises the steps of:
providing a luminol reagent including hydrogen peroxide; and
adding a catalase to the luminol reagent to decompose the hydrogen peroxide therein, and thereafter causing the reaction between the catalase and the hydrogen peroxide in the luminol reagent to be stopped.

Preferably, an inhibitor, by which the reaction between the catalase and the hydrogen peroxide in the luminol reagent is stopped, is added to the mixture of the catalase and the luminescent reagent. The inhibitor is preferably a sodium azide.

According to another aspect of the invention, a method for producing a reagent for use in the measurement of hydrogen peroxide in a sample comprises the steps of:
providing a luminol reagent including hydrogen peroxide; and
adding a catalase to the luminol reagent to decompose the hydrogen peroxide therein, the amount of the catalase being previously adjusted to a concentration of the catalase of 10 to 200 U/ml based on the total volume of the luminol reagent and the catalase in order to sufficiently decompose the hydrogen peroxide in the luminol reagent and not to affect the hydrogen peroxide in the sample when the luminol reagent is added to the sample.

According to another aspect of the invention, a method of hydrogen peroxide in a sample comprises the steps of:
providing a luminol reagent including hydrogen peroxide; and
causing the luminol reagent to pass through a column, in which an immobilized catalase is packed, to decompose the hydrogen peroxide in the luminol reagent.

According to another aspect of the invention, a method for producing a reagent for use in the measurement of hydrogen peroxide in a sample comprises the steps of:
providing a luminol reagent including a hydrogen peroxide; and
adding an immobilized catalase to the luminol reagent to decompose the hydrogen peroxide in the luminol reagent.

The immobilized catalase may comprise a catalase immobilized on a carrier which is formed by a reaction between a water insoluble carrier and a polyfunctional reagent.

According to another aspect of the invention, an apparatus for detecting light produced by the reaction between a luminol reagent and hydrogen peroxide in a sample in the presence of a catalyst for measuring the content of the hydrogen peroxide in the sample comprises:
a first vessel in which the luminol reagent is stored;
a second vessel in which the catalyst is stored;
housing means for defining an optically sealable hollow chamber, in which a cell is placed;
a column in which an immobilized catalase is packed, the immobilized catalase being active for decomposing hydrogen peroxide of the luminol reagent and the catalyst;
first means for injecting the luminol reagent and the catalyst from the first and second vessel into the cell in said housing chamber through the column; and
second means for measuring the quantity of light produced by the reaction between the luminol reagent and the hydrogen peroxide of the sample in the presence of the catalyst.

According further aspect of the invention, an apparatus for detecting light produced by the reaction between a luminol reagent and a hydrogen peroxide in a sample in the presence of a catalyst for measuring the content of the hydrogen peroxide in the sample comprises:
a first vessel in which the luminol reagent and an immobilized catalase active for decomposing hydrogen peroxide of the luminol reagent are stored;
a second vessel in which the catalyst and an immobilized catalase active for decomposing hydrogen peroxide of the catalyst are stored;
housing means for defining an optically sealable hollow chamber, in which a cell is placed;
first means for injecting the luminol reagent and the catalyst from the first and second vessel into the cell in said housing chamber; and
second means for measuring the quantity of light produced by the reaction between the luminol reagent and the hydrogen peroxide of the sample in the presence of the catalyst.

### BRIEF DESCRIPTION OF THE DRAWINGS

The present invention will be understood more fully the accompanying drawings of the preferred embodiments of the invention. The drawings are for explanation and understanding only.

In the drawings:
Figure 1 is a graph illustrating the quantity of emitted light versus the concentration of hydrogen peroxide when the reagent of the invention and the conventional reagent are used;
Figure 2 is a graph illustrating the quantity of light produced, which is an integrated value from 16 to 45 seconds after the reagent is added to the aqueous hydrogen peroxide, versus the concentration of hydrogen peroxide when the reagent of the invention and the conventional reagent are used;
Figure 3 is a graph illustrating the relationship between the quantity of emitted light and the concentration of hydrogen peroxide when reagents processed by various concentrations (0, 10, 100 and 500 U/ml) of catalase are used;
Figure 4 is a graph illustrating the relationship between the quantity of emitted light and the concentration of hydrogen peroxide when reagents processed by various concentrations (5, 10, 100, 200 and 250 U/ml) of catalase are used;
Figure 5 is a graph illustrating the quantity of emitted light, which is integrated values from 1 to 15 seconds and 16 to 30 seconds after the reagent is added to the aqeous hydrogen peroxide, versus the concentration of hydrogen peroxide when the reagent processed by 100 U/ml of catalase is used;
Figure 6 is a graph illustrating the quantity of emitted light due to a hydrogen peroxide versus time;
Figure 7 and 8 are graphs illustrating the quantity of emitted light obtained by using the reagent of the invention versus time;
Figure 9 is a graph illustrating the relationship between the quantity of emitted light and the concentration of hydrogen peroxide when a reagent of the invention is used, which reagent was caused to pass through a column in which immobilized catalase was packed;
Figure 10 is a schematic view of the preferred embodiment of an apparatus according to the invention, which has an immobilized catalase packed column;
Figure 11 is a graph illustrating the relationship between the quantity of emitted light and the concentration of hydrogen peroxide when a reagent of the invention is used, which reagent was processed by an immobilized catalase put into a nylon net bag; and
Figure 12 is a schematic view of the preferred embodiment of an apparatus according to the invention, which has a pair of tanks in which an immobilized catalase in a nylon net bag is disposed.

### DETAIL DESCRIPTION OF THE PREFERRED EMBODIMENT

The present invention resides in a new luminol reagent for use in the measurement of the content of hydrogen peroxide in a sample. The new reagent has little hydrogen peroxide, so that very small concentrations of hydrogen peroxide can be measured by using the new reagent.

According to the first preferred embodiment of the present invention, a luminol reagent is caused to react with a catalase to decompose the hydrogen peroxide therein. Thereafter, the reaction between the catalase and the hydrogen peroxide of the reagent is stopped. In order to cause the reaction to be stopped, an inhibitor, by which the reaction is stopped, is added to the mixture of the catalase and the reagent. The inhibitor is preferably sodium azide.

A catalase, the concentration of which is 100 U/ml based on the total volume, is added to a 2 X 10⁻⁷ mol/l of luminol serving as a luminescent reagent, and the luminol is allowed to react with the catalase for one hour at a room temperature. Thereafter, a sodium azide (NaN₃), the concentration of which is 0.01 mol/l based on the total volume, is added to the reaction product, so that a reagent of the invention is formed. The reagent of the invention reacts with hydrogen peroxide in the presence of a catalyst, such as a microperoxidase, to produce light. By measuring the produced light, the content of hydrogen peroxide can be determined.

When the concentration of the catalase is adjusted to be 10 to 200 U/ml, it is not required to remove the catalase from the reagent and to cause the reaction between the catalase and the hydrogen peroxide of the reagent to be stopped.

According to the second preferred embodiment of the present invention, a luminol reagent and/or a catalyst is caused to react with an immobilized catalase to decompose the hydrogen peroxide therein.

A water insoluble carrier is caused to react with a polyfunctional reagent, and thereafter a catalase is immobilized thereto. An aminoprophyl-CPG (controlled pore glass) and a glutaraldehyde are preferably used as the water insoluble carrier and the polyfunctional reagent, respectively. A 1 g of the aminopropyl-CPG is caused to react with 25 ml of a 2.5% of glutaraldehyde for three hours at room temperature to form aldehyde CPG. Thereafter, the aldehyde CPG is caused to react with 2 ml of a 100,000 U/ml of catalase solution for three hours at room temperature to produce an immobilized catalase. Using this immobilized catalase, the hydrogen peroxide(s) of the luminol solution and/or the catalyst solution is decomposed. That is, the luminol solution and/or the catalyst solution is caused to pass through a column, in which the immobilized catalase is packed, to form a reagent of the invention. Alternatively, the immobilized catalase may be put in a nylon net bag and added to solution(s) containing the luminol and/or the catalyst to form a reagent according to the invention.

The effectiveness of the present invention is illustrated by the following exampls.

### EXAMPLE I

A catalase was added to a buffer solution containing 2 X 10⁻⁷ mol/l luminol serving as a luminescent reagent and 0.2 mol/l carbonic acid to give a 100 U/ml of catalase based on the total volume, and the luminol was caused to react with the catalase for one hour at room temperature. Thereafter, sodium azide (NaN₃) was added to the reaction product to obtain a 0.01 mol/l sodium azide based on the total volume. This solution will be referred to as " a solution A". A 0.5 ml of the solution A and 0.5 ml of an aqueous solution containing a 5 X 10⁻⁶ mol/l microperoxidase serving as a catalyst were added to 0.1ml of solutions having various concentrations (10⁻³ to 10⁻⁹ mol/l) of hydrogen peroxide to be tested. The quantity of light produced by each of the reactions between the luminol and the hydrogen peroxide in the presence of the microperoxidase was measured by a luminometer UPD-8000 (made by KABUSHIKI KAISHA MEIDENSHA) as an integrated value for 15 seconds after mixing.

As a reference, 0.5 ml of an aqueous solution containing a 2 X 10⁻⁷ mol/l luminol which was not processed by the catalase, and 0.5 ml of a 5 X 10⁻⁶ mol/l microperoxidase were added to 0.1 ml of solutions having various concentrations (10⁻³ to 10⁻⁹ mol/l) of hydrogen peroxides. The quantity of light produced by each of the respective reactions was measured in a similar manner to Example I (Reference I).

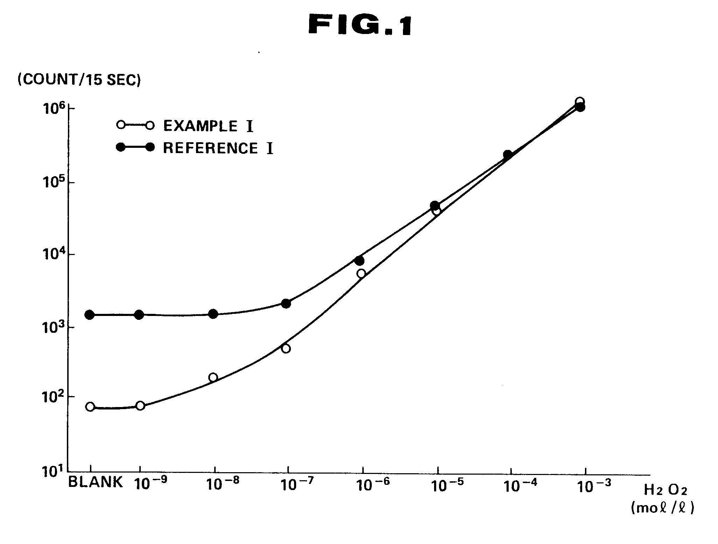

Figure 1 shows the relationship between the concentration of hydrogen peroxide and the quantity of light produced by each of the respective reactions.

In the case of Reference I, the proportion of the hydrogen peroxide included in the luminol to the aqueous hydrogen peroxide which was actually added to the luminol and the microperoxidase is relatively high when the concentration of the aqueous hydrogen peroxide is relatively low, such as less than 10⁻⁷ mol/l. As a result, the quantity of emitted light is much greater than that of light produced by the aqueous hydrogen peroxide which was actually added to the luminol and the microperoxidase. That is, the background light greatly affected the measured value when the concentration of hydrogen peroxide was less than 10⁻⁷ mol/l, so that it was difficult to measure the actual content of hydrogen peroxide in such a low concentration. On the other hand, in the case of Example I, the background light hardly affected the measured value when the concentration of hydrogen peroxide was about 10⁻⁸ mol/l. Therefore, it was found that the content of hydrogen peroxide can be accurately measured even in low concentration, up to about 10⁻⁸ mol/l, by using the reagent of the invention.

### EXAMPLE II

A 0.5 ml of the solution A in Example I and 0.5 ml of an aqueous solution containing a 6 X 10⁻³ mol/l potassium ferricyanide serving as a catalyst were added to 0.1 ml of solutions having various concentrations (10⁻⁴ to 10⁻⁹ mol/l) of hydrogen peroxide. After 15 seconds, the quantity of light produced by the reaction between the luminol and the hydrogen peroxide in the presence of the potassium ferricyanide was measured for 30 seconds by a luminometer UPD-8000 to obtain an integrated value thereof.

In order to compare with Example II, 0.5 ml of a 2 X 10⁻⁷ mol/l of luminol solution which was not processed by the catalase, and 0.5 ml of a 6 X 10⁻³ mol/l of potassium ferricyanide solution were added to 0.1 ml of solutions havbing various concentrations (10⁻⁴ to 10⁻⁹ mol/l) of hydrogen peroxides. The quantity of light produced by the respective reactions was measured in a similar manner to Example II (Reference II).

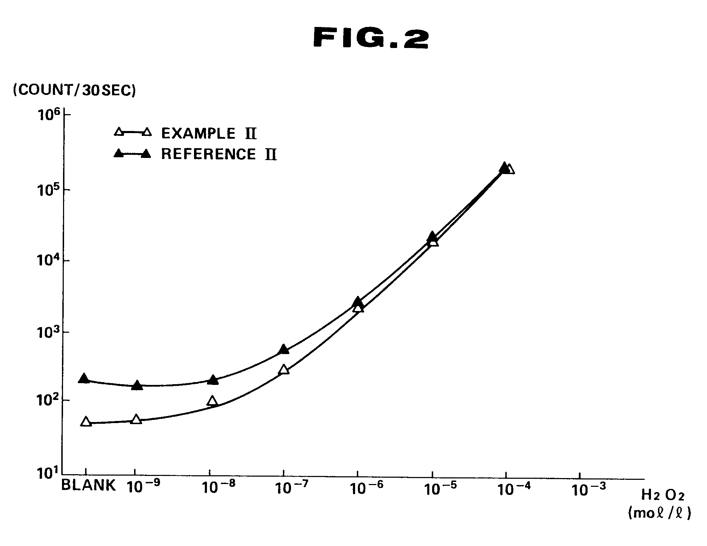

Fig. 2 shows the relationship between the concentration of hydrogen peroxide and the quantity of light produced by each of the aforementioned reactions.

As shown in Fig. 2, similar results to Example I were obtained when a predetermined time passed after the solution A was added to the aqueous hydrogen peroxide. Therefore, it was found that the reagent of the invention can be used when the reagent and the sample are pipetted not only by means of an automatic pipetting device but also manually, since about ten seconds are required until a cell is set at a measuring position in a measuring apparatus after the reagent and the sample are injected into the cell.

### EXAMPLE III

In order to determine the optimum concentration of catalase, the following experiment was carried out.

A catalase was added to an aqueous solution containing 2 X 10⁻⁷ mol/l luminol and 5 X 10⁻⁶ mol/1 microperoxidase to obtain solutions containing 500 U/ml, 100 U/ml and 1U/ml catalases respectively, and they were permitted to stand for one hour. Thereafter, 0.5 ml of the respective solutions were added to 0.1 ml of aqueous solutions containing 10⁻³ to 10⁻⁹ mol/l hydrogen peroxide. The quantity of light produced by the reaction between the luminol and the hydrogen peroxide in the presence of the microperoxidase in the respective samples was measured by the luminometer UPD-8000.

For reference, 0.5 ml of an aqueous solution containing 2 X 10⁻⁷ mol/l of luminol, and 0.5 ml of an aqueous solution containing 5 X 10⁻⁶ mol/l of microperoxidase were added to 0.1 ml of aqueous solutions containing 10⁻³ to 10⁻⁹ mol/l hydrogen peroxides. The quantity of light produced by the respective reactions was measured in a similar manner to Example III (Reference III).

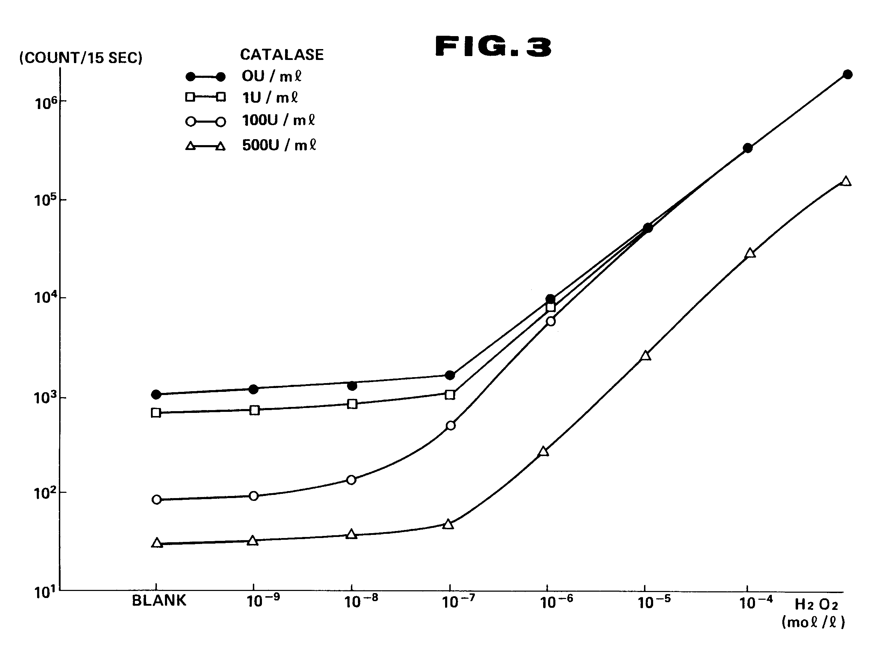

Fig. 3 shows the relationship between the concentrations of hydrogen peroxides and the quantities of light produced by the respective reactions. When the concentration of catalase is 500 U/ml, blank value which is the quantity of emitted light when no hydrogen peroxide is contained in the sample can be decreased. However, since the amount of catalase was excessive in the solution, the hydrogen peroxide to be tested was also decomposed, so that the emitted light values of samples containing hydrogen peroxide were also decreased. On the other hand, when the concentration of catalase is 1 U/ml, the hydrogen peroxide of the luminol is not sufficiently decomposed to cause the blank value to be effectively decreased, since the concentration of catalase is too low. In this case, it was found that the optimum concentration of catalase was 100 U/ml.

### EXAMPLE IV

In order to embody the optimum concentration of catalase to be added to the sample, a catalase was added to a solution containing 2 X 10⁻⁷ mol/l luminol and 5 X 10⁻⁶ mol/l microperoxidase to give solutions having 200 U/ml, 100 U/ml and 10 U/ml catalase, and they were allowed to stand for one hour. Thereafter, 0.5 ml of the respective solutions were added to 0.1 ml of aqueous solutions having various concentrations (10⁻³ to 10⁻⁹ mol/l) of hydrogen peroxide, and the quantity of light produced by the respective reactions therebetween was measured by the luminometer UPD-8000.

In order to compare with Example III, catalase was added to to a solution containing 2 X 10⁻⁷ mol/l luminol and 5 X 10⁻⁶ mol/l microperoxidase to give solutions containing 250 U/ml (Reference IV-I) and 5 U/ml (Reference IV-II) catalase, which were allowed to stand for one hour. Thereafter, 0.5 ml of these solutions were respectively mixed with 0.1 ml samples containing 10⁻³ to 10⁻⁹ mol/l hydrogen peroxide, and the quantity of light produced by the respective reactions therebetween was measured in the same manner.

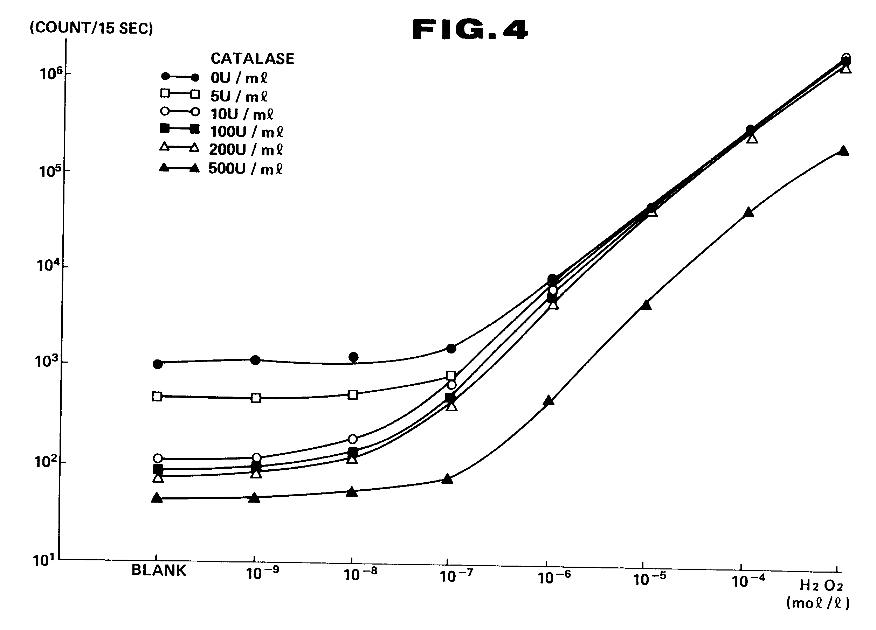

These results are shown in Fig. 4. As shown in Fig. 4, when the concentration of catalase are 200 U/ml, 100 U/ml and 10 U/ml, the blank value can be sufficiently decreased to improve the sensitivity of the measurement, and the hydrogen peroxide of the sample to be tested is not decomposed by excess catalase. When the concentration of catalase is greater than 200 U/ml, i.e. when it is 250 U/ml, the value of blank can be. decreased, however, since the amount of catalase in the solution is excessive, even the hydrogen peroxide to be tested is decomposed by the excess catalase, and all measured value are decreased. On the other hand, when the concentration of catalase is 5 U/ml, the hydrogen peroxide of the luminol is not sufficiently decomposed so that the blank value can not be effectively decreased, since the concentration of catalase was too low. Therefore, it was found that the optimum concentration of catalase was from 10 U/ml to 200 U/ml.

### EXAMPLE V

The optimum measuring time, in which the aforementioned reagent is used in an automatic pipetting system, was determined by the following experiments.

A catalase was added to a solution containing 2 X 10⁻⁷ mol/l luminol and 5 X 10⁻⁶ mol/l microperoxidase so that the concentration of the catalase was 100 U/ml, and was allowed to stand for one hour. Thereafter, 0.5 ml of this solution and 0.1 ml of aqueous hydrogen peroxide were injected into the cell by means of the automatic pipetting system, and the quantity of light produced by the reaction therebetween was measured by the luminometer UPD-8000 to obtain an integrated value thereof for 15 seconds after mixing.

As a reference, the quantity of emitted light produced by the aforementioned reaction was measured during a 15 second period commencing 15 seconds after mixing in the same manner, to obtain an integrated value thereof (Reference V).

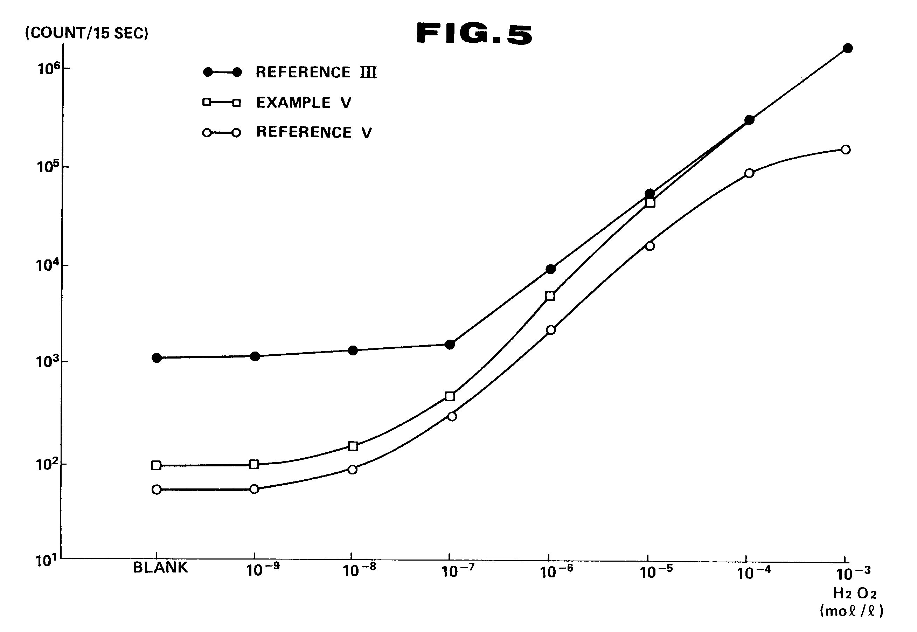

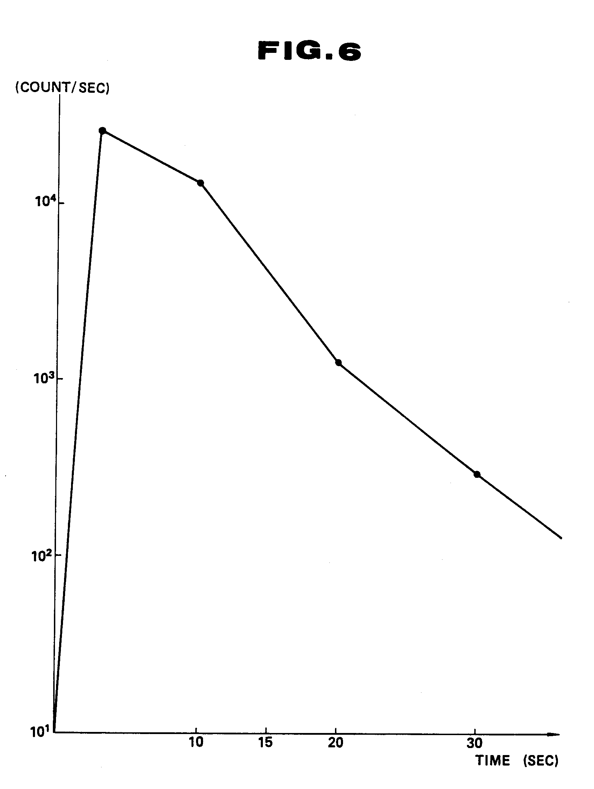

The results of the above measurements are shown in Fig. 5. For reference, the result of Reference III is also represented in Fig. 5. In the case of Reference V, the blank value was decreased, but so were all of the other measured values, since the quantity of emitted light reaches its peak two or three seconds after the reagent is added to the sample in a typical luminescent phenomenon due to a hydrogen peroxide (See Fig. 6). Therefore, it was found that the quantity of emitted light was preferably measured for 15 seconds after mixing by means of an automatic pipetting device when the reagent of the invention was used.

### EXAMPLE VI

In order to examine the stability of the reagent of the invention, the following experiment was carried out.

A catalase was added to a solution containing 2 X 10⁻⁷ mol/l luminol and 6 X 10⁻³ mol/l potassium ferricyanide, and the quantity of emitted light (as an integrated value for 15 seconds) was measured by the luminometer UPD-8000 immediately, half, 1, 5, 10, 15 20 and 24 hours after mixing.

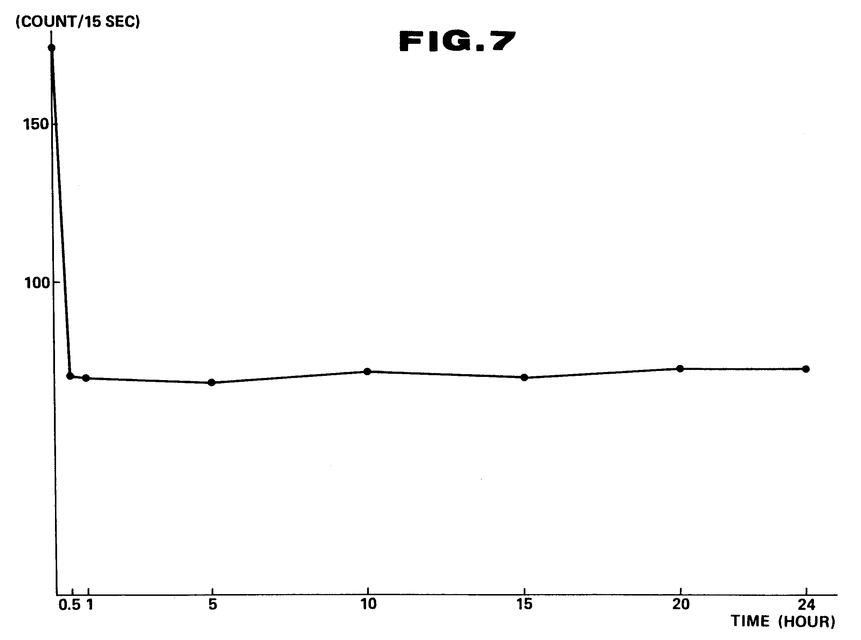

These result are shown in Fig. 7. As shown in Fig. 7, it was found that the reagent of the invention was stable regardless of elapsed time.

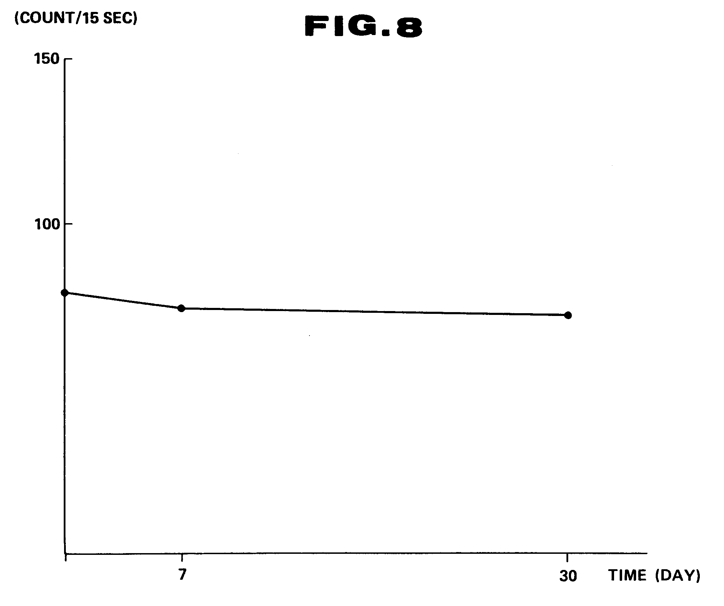

In addition, the quantity of emitted light produced by the same mixture are also measured one week and one month after adding. As shown in Fig. 8, it was found that the reagent of the invention was still stable after one month.

### EXAMPLE VII

A polyfunctional reagent was caused to react with water insoluble carrier, and thereafter a catalase was attached thereto so as to immobilize the catalase. An aminopropyl-CPG (porous glass, diameter of pore; 120-200 mesh, made by ELECTRO-NUCLEONICS INC) and a glutaraldehyde were used as the water insoluble carrier and the polyfunctional reagent, respectively. 1g of the aminopropyl-CPG was immersed in 25 ml of a buffer solution containing 2.5 % glutaraldehyde and 0.01 % phosphoric acid for three hours at room temperature to form an aldehyde CPG. Thereafter, the aldehyde CPG was caused to react with 2 ml of a buffer solution containing 100,000 U/ml catalase and 0.01 % phosphoric acid for three hours at room temperature to produce an immobilized catalase.

A luminol serving as a luminescent reagent was then caused to pass through a columun in which the aforementioned immobilized catalase was packed. A 0.5 ml of this solution and 0.5 ml of microperoxidase were added to 0.1 ml of solutions having various concentrations (10⁻³ to 10⁻⁹ mol/l) of hydrogen peroxide. The quantity of emitted light produced by the respective reactions was measured as integrated values for 15 seconds by means of the luminometer.

As a reference, luminol which was not processed by the immobilized catalase was added to 0.1 ml of each of the same hydrogen peroxides, and the quantity of emitted light was measured (Reference VII).

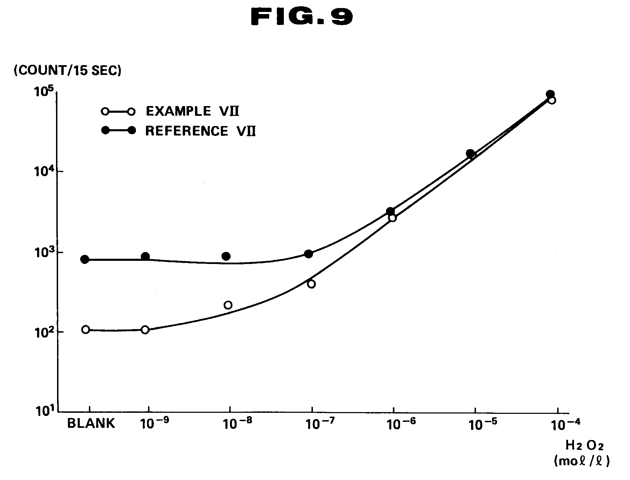

These results are shown in Fig. 9. In the case of Reference VII, the proportion of the hydrogen peroxide of the luminol to the hydrogen peroxide which is added with the sample to be tested is relatively high when the concentration of hydrogen peroxide in the sample is relatively low, such as less than 10⁻⁷ mol/l. As a result, the quantity of emitted light produced by the hydrogen peroxide already contained in the reaction solution is much greater than that produced by the hydrogen peroxide of the sample. That is, the background light greatly affects the measured value when the concentration of hydrogen peroxide in the sample is less than 10⁻⁷ mol/l, so that it is difficult to measure the actual content of hydrogen peroxide in such low concentrations. On the other hand, in the case of Example VII, the background light hardly affects the measured value even when the concentration of hydrogen peroxide is about 10⁻⁸ mol/l. Therefore, it was found that the content of hydrogen peroxide can be measured in concentrations of hydrogen peroxide as little as about 10⁻⁸ mol/l, by using the reagent of the invention.

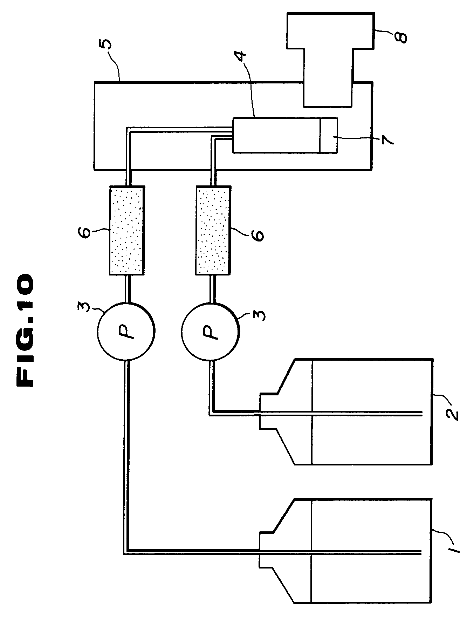

According to the present invention, the method of the invention can be performed by using an apparatus shown in Fig. 10. A luminescent reagent, such as luminol, and a catalyst, such as a microperoxidase, are stored in tanks 1 and 2, respectively. The luminescent reagent and the catalyst are sucked up by means of pumps 3 and introduced into a cell 4 within a black box 5 via columns 6 in which the immobilized catalases are packed. The light produced from a sample 7 including the luminescent reagent and the catalyst is detected by a photomultiplier tube 8.

### EXAMPLE VIII

The immobilized catalase which was produced in a similar manner to Example VII was put into a nylon net bag. The immobilized catalases in the nylon net bags were immersed in 100 ml of luminol solution and 100 ml of microperoxidase solution, respectively. Thereafter, 0.5 ml of the luminol solution and 0.5 ml of the microperoxidase solution were added to 0.1 ml of solutions having various concentrations (10⁻⁴ to 10⁻⁹ mol/l) of hydrogen peroxide. The quantity produced by the respective reactions was measured by luminometer UPD-8000.

As reference, by using a luminol and a microperoxidase which were not processed by the immobilized catalase, the quantity of light produced by the respective reactions was measured in the same manner.

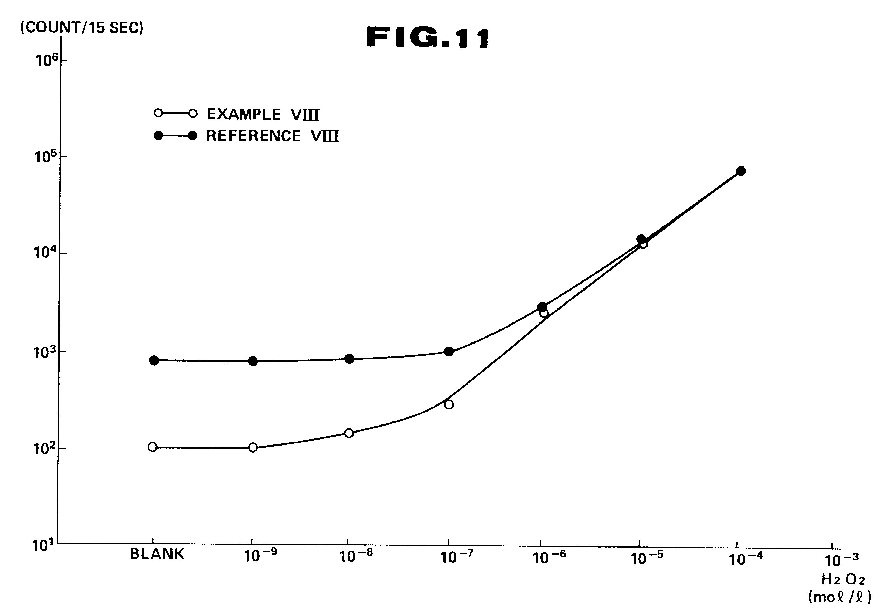

These results are shown in Fig. 11. As shown in Fig. 11, it was found that the same results as Example VII were also obtained by this example.

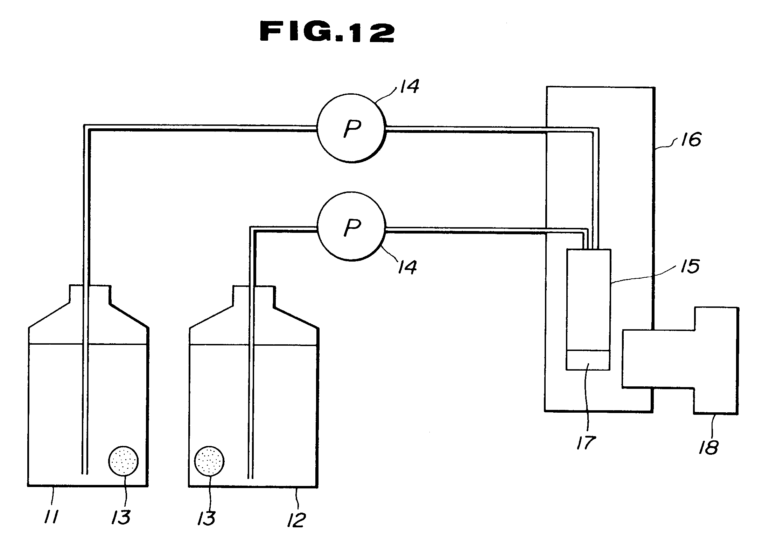

According to the present invention, the method of the invention can be performed by using an apparatus shown in Fig. 12. The luminescent reagent and the catalyst are stored in tanks 11 and 12, respectively. The immobilized catalases 13 in the nylon net bags are also disposed in the respective tanks 11 and 12. The luminescent reagent and the catalyst are sucked up by means of pumps 14 and introduced into a cell 15 within a black box 16. The light produced from a sample 17 including the luminescent reagent and the catalyst is detected by a photomultiplier tube 18.

## Claims

1. A process for measuring the content of hydrogen peroxide in a sample comprising:
mixing a luminol reagent including hydrogen peroxide with a catalase to decompose the hydrogen peroxide of the reagent;
adding an inhibitor, by which the reaction between the catalase and the hydrogen peroxide is stopped, to the mixure of the catalase and the luminol reagent;
adding said luminol reagent to said sample; and
measuring the content of hydrogen peroxide in said sample.

2. A process as set forth in claim 1, wherein said inhibitor is sodium azide.

3. A process for measuring the content of hydrogen peroxide in a sample comprising:
mixing a luminol reagent including hydrogen peroxide which a catalase, the concentration of which is 10 to 200 U/ml based on the total volume, to decompose the hydrogen peroxide of the luminol reagent;
adding said luminol reagent to said sample; and
measuring the content of hydrogen peroxide in said sample.

4. A process for measuring the content of hydrogen peroxide in a sample comprising:
bringing a luminol reagent including hydrogen peroxide into contact with an immobilized catalase to decompose the hydrogen peroxide in the luminol reagent;
adding said luminol reagent to said sample; and
measuring the content of hydrogen peroxide in said sample.

5. A process as set forth in claim 4, wherein said luminol reagent is caused to pass through a column, in which said immobilized catalase is packed, to come into contact with said immobilized catalase.

6. A process as set forth in claim 4, wherein said immobilized catalase comprises a catalase which is immobilized on a carrier formed by a reaction between a water insoluble carrier and a polyfunctional reagent.

7. A reagent for use in the measurement of hydrogen peroxide in a sample, which comprises a luminol reagent and a catalase, the concentration of which is 10 to 200 U/ml based on the total volume.

8. A reagent for use in the measurement of hydrogen peroxide in a sample, which comprises a luminol reagent and an immobilized catalase.

9. A reagent as set forth in claim 8, wherein said immobilized catalase comprises a catalase immobilized on a carrier which is formed by a reaction between a water insoluble carrier and a polyfunctional reagent.

10. A reagent as set forth in claim 9, wherein said water insoluble carrier is aminopropyl-CPG.

11. A reagent as set forth in claim 9, wherein said polyfunctional reagent is glutaraldehyde.

12. A method for producing a reagent for use in the measurement of hydrogen peroxide in a sample, which comprises the steps of:
providing a luminol reagent including hydrogen peroxide; and
adding a catalase to said luminol reagent to decompose the hydrogen peroxide therein, and thereafter removing said catalase remaining in said luminol reagent.

13. A method for producing a reagent for use in the measurement of hydrogen peroxide in a sample, which comprises the steps of:
providing a luminol reagent including hydrogen peroxide; and
adding a catalase to said luminol reagent to decompose the hydrogen peroxide therein, and thereafter causing the reaction between the catalase and the hydrogen peroxide in the luminol reagent to be stopped.

14. A method as set forth in claim 13, wherein an inhibitor, by which said reaction between the catalase and the hydrogen peroxide in the luminol reagent is stopped is added to the mixture of the catalase and the luminol reagent.

15. A method as set forth in claim 14, wherein said inhibitor is sodium azide.

16. A method for producing a reagent for use in the measurement of hydrogen peroxide in a sample, which comprises the steps of:
providing a luminol reagent including hydrogen peroxide; and
adding a catalase to said luminol reagent to decompose the hydrogen peroxide therein, the amount of said catalase being previously adjusted to a concentration of said catalase of 10 to 200 U/ml based on the total volume of the luminol reagent and the catalase in order to sufficiently decompose the hydrogen peroxide in the luminol reagent and not to affect the hydrogen peroxide in said sample when said luminol reagent is added to said sample.

17. A method for producing a reagent for use in the measurement of hydrogen peroxide in a sample, which comprises the steps of:
providing a luminol reagent including reagent including hydrogen peroxide; and
causing said luminol reagent to pass through a column, in which an immobilized catalase is packed, to decompose the hydrogen peroxide in the luminol reagent.

18. A method as set forth in claim 17, wherein said immobilized catalase comprises a catalase immobilized on a carrier which is formed by a reaction between a water insoluble carrier and a polyfunctional reagent.

19. A method for producing a reagent for use in the measurement of hydrogen peroxide in a sample, which comprises the steps of:
providing a luminol reagent including hydrogen peroxide; and
adding an immobilized catalase to said luminol reagent to decompose the hydrogen peroxide in the luminol reagent.

20. A method as set forth in claim 19, wherein said immobilized catalase comprises a catalase immobilized on a carrier which is formed by a reaction between a water insoluble carrier and a polyfunctional reagent.

21. An apparatus for detecting light produced by the reaction between a luminol reagent and hydrogen peroxide in a sample in the presence of a catalyst for measuring the content of the hydrogen peroxide in the sample, which comprises:
a first vessel in which said luminol reagent is stored;
a second vessel in which said catalyst is stored;
housing means for defining an optically sealable hollow chamber, in which a cell is placed;
a column in which an immobilized catalase is packed, said immobilized catalase being active for decomposing hydrogen peroxide of said luminol reagent and said catalyst;
first means for injecting said luminol reagent and said catalyst from said first and second vessel into said cell in said housing chamber through said column; and
second means for measuring the quantity of light produced by the reaction between said luminol reagent and said hydrogen peroxide of said sample in the presence of said catalyst.

22. An apparatus for detecting light produced by the reaction between a luminol reagent and hydrogen peroxide in a sample in the presence of a catalyst for measuring the content of the hydrogen peroxide in the sample, which comprises:
a first vessel in which said luminol reagent and an immobilized catalase active for decomposing hydrogen peroxide of said luminol reagent are stored;
a second vessel in which said catalyst and an immobilized catalase active for decomposing hydrogen peroxide of said catalyst are stored;
housing means for defining an optically sealable hollow chamber, in which a cell is placed;
first means for injecting said luminol reagent and said catalyst from said first and second vessel into said cell in said housing chamber; and
second means for measuring the quantity of light produced by the reaction between said luminol reagent and said hydrogen peroxide of said sample in the presence of said catalyst.

## Patentansprüche

1. Verfahren zur Messung des Gehaltes von Wasserstoffperoxid in einer Probe, umfassend:
Mischen eines Luminolreagens, das Wasserstoffperoxid einschließt, mit einer Katalase zur Zersetzung des Wasserstoffperoxids des Luminolreagens;
Zugabe eines Inhibitors, durch welchen die Reaktion zwischen der Katalase und dem Wasserstoffperoxid abgestoppt wird, zu der Mischung der Katalase und des Luminolreagens;
Zugabe dieses Luminolreagens zu dieser Probe; und
Messung des Gehaltes von Wasserstoffperoxid in dieser Probe.

2. Verfahren nach Anspruch 1, worin dieser Inhibitor Natriumazid ist.

3. Verfahren zur Messung des Gehaltes von Wasserstoffperoxid in einer Probe, umfassend:
Mischen eines Luminolreagens, das Wasserstoffperoxid einschließt, mit einer Katalase, deren Konzentration 10 bis 200 E/ml, bezogen auf das Gesamtvolumen, beträgt, zur Zersetzung des Wasserstoffperoxids des Luminolreagens;
Zugabe dieses Luminolreagens zu dieser Probe; und
Messung des Gehaltes von Wasserstoffperoxid in dieser Probe.

4. Verfahren zur Messung des Gehaltes von Wasserstoffperoxid in einer Probe, umfassend:
Inkontaktbringen eines Luminolreagens, das Wasserstoffperoxid einschließt, mit einer immobilisierten Katalase zur Zersetzung des Wasserstoffperoxids in dem Luminolreagens;
Zugabe dieses Luminolreagens zu dieser Probe; und
Messung des Gehaltes von Wasserstoffperoxid in dieser Probe.

5. Verfahren nach Anspruch 4, worin dieses Luminolreagens zum Durchtreten durch eine Säule, in welcher die immobilisierte Katalase gepackt ist, veranlaßt wird, um mit dieser immobilisierten Katalase in Kontakt zu kommen.

6. Verfahren nach Anspruch 4, worin diese immobilisierte Katalase eine Katalase umfaßt, die auf einem durch Reaktion zwischen einem wasserunlöslichen Träger und einem polyfunktionellen Reagens gebildeten Träger immobilisiert ist.

7. Reagens zur Anwendung bei der Messung von Wasserstoffperoxid in einer Probe, welches ein Luminolreagens und eine Katalase umfaßt, deren Konzentration 10 bis 200 E/ml, bezogen auf das Gesamtvolumen, beträgt.

8. Reagens zur Anwendung bei der Messung von Wasserstoffperoxid in einer Probe, welches ein Luminolreagens und eine immobilisierte Katalase umfaßt.

9. Reagens nach Anspruch 8, worin diese immobilisierte Katalase eine Katalase umfaßt, die auf einem durch Reaktion zwischen einem wasserunlöslichen Träger und einem polyfunktionellen Reagens gebildeten Träger immobilisiert ist.

10. Reagens nach Anspruch 9, worin dieser wasserunlösliche Träger Amminopropyl-CPG ist.

11. Reagens nach Anspruch 9, worin dieses polyfumktionelle Reagens Glutaraldehyd ist.

12. Verfahren zur Herstellung eines Reagens zur Anwendung bei der Messung von Wasserstoffperoxid in einer Probe, welches die Stufen umfaßt von:
Bereitstellung eines Luminolreagens, das Wasserstoffperoxid einschließt; und
Zugabe einer Katalase zu diesem Luminolreagens zur Zersetzung des Wasserstoffperoxids hierin, und danach Entfernung dieser in diesem Luminolreagens zurückbleibenden Katalase.

13. Verfahren zur Herstellung eines Reagens zur Anwendung bei der Messung von Wasserstoffperoxid in einer Probe, welches die Stufen umfaßt von:
Bereitstellung eines Luminolreagens, das Wasserstoffperoxid einschließt; und
Zugabe einer Katalase zu diesem Luminolreagens zur Zersetzung des Wasserstoffperoxids hierin, und danach Bewirken des Abstoppens der Reaktion zwischen der Katalase und dem Wasserstoffperoxid in dem Luminolreagens.

14. Verfahren nach Anspruch 13, worin ein Inhibitor, durch den diese Reaktion zwischen der Katalase und dem Wasserstoffperoxid abgestoppt wird, zu der Mischung der Katalase und des Luminolreagens zugesetzt wird.

15. Verfahren nach Anspruch 14, worin dieser Inhibitor Natriumazid ist.

16. Verfahren zur Herstellung eines Reagens zur Anwendung bei der Messung von Wasserstoffperoxid in einer Probe, welches die Stufen umfaßt von:
Bereitstellung eines Luminolreagens, das Wasserstoffperoxid einschließt; und
Zugabe einer Katalase zu diesem Luminolreagens zur Zersetzung des Wasserstoffperoxids hierin, wobei die Menge dieser Katalase zuvor auf eine Konzentration dieser Katalase von 10 bis 200 E/ml, bezogen auf das Gesamtvolumen des Luminolreagens und der Katalase, eingestellt wurde, um das Wasserstoffperoxid in dem Luminolreagens ausreichend zu zersetzen und das Wasserstoffperoxid in dieser Probe nicht zu beeinflussen, wenn dieses Luminolreagens zu dieser Probe zugesetzt wird.

17. Verfahren zur Herstellung eines Reagens zur Anwendung bei der Messung von Wasserstoffperoxid in einer Probe, welches die Stufen umfaßt von:
Bereitstellung eines Luminolreagens, das Wasserstoffperoxid einschließt; und
Veranlassen des Durchtretens dieses Luminolreagens durch eine Säule, in welcher eine immobilisierte Katalase gepackt ist, zur Zersetzung des Wasserstoffperoxids in dem Luminolreagens.

18. Verfahren nach Anspruch 17, worin diese immobilisierte Katalase eine Katalase umfaßt, die auf einem durch Reaktion zwischen einem wasserunlöslichen Träger und einem polyfunktionellen Reagens gebildeten Träger immobilisiert ist.

19. Verfahren zur Herstellung eines Reagens zur Anwendung bei der Messung von Wasserstoffperoxid in einer Probe, welches die Stufen umfaßt von:
Bereitstellung eines Luminolreagens, das Wasserstoffperoxid einschließt; und
Zugabe einer immobilisierten Katalase zu diesem Luminolreagens zur Zersetzung des Wasserstoffperoxids in dem Luminolreagens.

20. Verfahren nach Anspruch 19, worin diese immobilisierte Katalase eine Katalase umfaßt, die auf einem durch Reaktion zwischen einem wasserunlöslichen Träger und einem polyfunktionellen Reagens gebildeten Träger immobilisiert ist.

21. Vorrichtung zum Nachweis von Licht, das durch Reaktion zwischen einem Luminolreagens und Wasserstoffperoxid in einer Probe in Anwesenheit eines Katalysators erzeugt wird, zur Messung des Gehaltes des Wasserstoffperoxids in der Probe, welche umfaßt:
einen ersten Behälter, in dem dieses Luminolreagens aufbewahrt wird;
einen zweiten Behälter, in dem dieser Katalysator aufbewahrt wird;
Gehäuseeinrichtungen zur Begrenzung einer optisch abgedichteten Hohlkammer, in der eine Zelle angeordnet ist;
eine Säule, in der eine immobilisierte Katalase gepackt ist, wobei diese immobiliserte Katalase zur Zersetzung von Wasserstoffperoxid dieses Luminolreagens und dieses Katalysators aktiv ist;
erste Einrichtungen zum Injizieren dieses Luminolreagens und dieses Katalysators aus diesem ersten und zweiten Behälter in diese Zelle in dieser Gehäusekammer durch diese Säule; und
zweite Einrichtungen zum Messen der Menge an Licht, welche durch die Reaktion zwischen diesem Luminolreagens und diesem Wasserstoffperoxid dieser Probe in Anwesenheit dieses Katalysators erzeugt wird.

22. Vorrichtung zum Nachweis von Licht, das durch Reaktion zwischen einem Luminolreagens und Wasserstoffperoxid in einer Probe in Anwesenheit eines Katalysators erzeugt wird, zur Messung des Gehaltes des Wasserstoffperoxids in der Probe, welche umfaßt:
einen ersten Behälter, in dem dieses Luminolreagens und eine zur Zersetzung von Wasserstoffperoxid dieses Luminolreagens aktive, immobilisierte Katalase aufbewahrt werden;
einen zweiten Behälter, in dem dieser Katalysator und eine zur Zersetzung von Wasserstoffperoxid dieses Katalysators aktive, immobilisierte Katalase aufbewahrt werden;
Gehäuseeinrichtungen zur Begrenzung einer optisch abgedichteten Hohlkammer, in der eine Zelle angeordnet ist;
erste Einrichtungen zum Injizieren dieses Luminolreagens und dieses Katalysators aus diesem ersten und zweiten Behälter in diese Zelle in dieser Gehäusekammer; und
zweite Einrichtungen zum Messen der Menge an Licht, welche durch die Reaktion zwischen diesem Luminolreagens und diesem Wasserstoffperoxid dieser Probe in Anwesenheit dieses Katalysators erzeugt wird.

## Revendications

1. Procédé pour mesurer la teneur de péroxide d'hydrogène dans un échantillon, comprenant les étapes suivantes:
- on mélange un réactif au luminol qui comprend du péroxide d'hydrogène avec une catalase pour décomposer le péroxide d'hydrogène du réactif;
- on ajoute un inhibiteur, au moyen duquel la réaction entre la catalase et le péroxide d'hydrogène est arrêtée, au mélange de catalase et de réactif de luminol;
- on ajoute ledit réactif de luminol audit échantillon; et
- on mesure la teneur de péroxide d'hydrogène dans ledit échantillon.

2. Procédé selon la revendication 1, caractérisé en ce que ledit inhibiteur est de l'acide de sodium.

3. Procédé pour mesurer la teneur de péroxide d'hydrogène dans un échantillon, comprenant les étapes suivantes:
- on mélange un réactif au luminol qui comprend du péroxide d'hydrogène avec une catalase, dont la concentration est de 10 à 200 U/ml sur la base du volume total afin de décomposer le péroxide d'hydrogène du réactif au luminol;
- on ajoute ledit réactif au luminol audit échantillon;
- on mesure la teneur de péroxide d'hydrogène dans ledit échantillon.

4. Procédé pour mesurer la teneur de péroxide d'hydrogène dans un échantillon, comprenant les étapes suivantes:
- on amène un réactif au luminol qui comprend du péroxide d'hydrogène en contact avec une catalase immobilisée pour décomposer le péroxide d'hydrogène dans le réactif au luminol;
- on ajoute ledit réactif au luminol audit échantillon; et
- on mesure la teneur de péroxide d'hydrogène dans ledit échantillon.

5. Procédé selon la revendication 4, caractérisé en ce que l'on fait passer ledit réactif au luminol à travers une colonne garnie avec ladite catalase immobilisée, afin de venir en contact avec ladite catalase immobilisée.

6. Procédé selon la revendication 4, caractérisé en ce que ladite catalase immobilisée comprend une catalase qui est immobilisée sur un support formé par une réaction entre un support insoluble à l'eau et un réactif polyfonctionnel.

7. Réactif destiné à être utilisé pour la mesure de péroxide d'hydrogène dans un échantillon, qui comprend un réactif au luminol et une catalase, dont la concentration est de 10 à 200 U/ml, basée sur le volume total.

8. Réactif destiné à être utilisé pour la mesure de péroxide d'hydrogène dans un échantillon, qui comprend un réactif au luminol et une catalase immobilisée.

9. Réactif selon la revendication 8, caractérisé en ce que ladite catalase immobilisée comprend une catalase immobilisée sur un support qui est formé par une réaction entre un support insoluble à l'eau et un réactif polyfonctionnel.

10. Réactif selon la revendication 9, caractérisé en ce que ledit support insoluble à l'eau est de l'aminopropyl-CPG.

11. Réactif selon la revendication 9, caractérisé en ce que ledit réactif polyfonctionnel du glutaraldéhyde.

12. Procédé pour produire un réactif destiné à être utilisé pour la mesure de péroxide d'hydrogène dans un échantillon, comprenant les étapes suivantes:
- on prend un réactif au luminol comprenant du péroxide d'hydrogène; et
- on ajoute une catalase audit réactif au luminol pour décomposer le péroxide d'hydrogène dans celui-ci, et on enlève ensuite ladite catalase qui reste dans le réactif au luminol.

13. Procédé pour produire un réactif destiné à être utilisé pour la mesure de péroxide d'hydrogène dans un échantillon, qui comprend les étapes suivantes:
- on prend un réactif au luminol comprenant du péroxide d'hydrogène; et
- on ajoute une catalase audit réactif au luminol pour décomposer le péroxide d'hydrogène dans celui-ci, et on provoque ensuite l'arrêt de la réaction entre la catalase et le péroxide d'hydrogène dans le réactif au luminol.

14. Procédé selon la revendication 13, caractérisé en ce qu'on ajoute au mélange de la catalase et du réactif au luminol un inhibiteur, au moyen duquel ladite réaction entre la catalase et le péroxide d'hydrogène dans le réactif au luminol est arrêtée.

15. Procédé selon la revendication 14, caractérisé en ce que ledit inhibiteur est de l'acide de sodium.

16. Procédé pour produire un réactif destiné à être utilisé pour la mesure de péroxide d'hydrogène dans un échantillon, comprenant les étapes suivantes:
- on prend un réactif au luminol comprenant du péroxide d'hydrogène; et
- on ajoute une catalase audit réactif au luminol pour décomposer le péroxide d'hydrogène dans celui-ci, la quantité de ladite catalase étant auparavant ajustée à une concentration de catalase de 10 à 200U/ml basée sur le volume total du réactif au luminol et de la catalase, afin de décomposer suffisamment le péroxide d'hydrogène dans le réactif au luminol et de ne pas affecter le péroxide d'hydrogène dans ledit échantillon lorsqu'on ajoute ledit réactif au luminol audit échantillon.

17. Procédé pour produire un réactif destiné à être utilisé pour la mesure de péroxide d'hydrogène dans un échantillon, qui comprend les étapes suivantes:
- on prend un réactif au luminol comprenant du péroxide d'hydrogène; et
- on amène ledit réactif au luminol à passer à travers une colonne qui est garnie avec une catalase immobilisée, afin de décomposer le péroxide d'hydrogène dans le réactif au luminol.

18. Procédé selon la revendication 17, caractérisé en ce que ladite catalase immobilisée comprend une catalase immobilisée sur un support qui est formé par une réaction entre un support insoluble à l'eau et un réactif polyfonctionnel.

19. Procédé pour produire un réactif destiné à être utilisé pour la mesure de péroxide d'hydrogène dans un échantillon, comprenant les étapes suivantes:
- on prend un réactif au luminol comprenant du péroxide d'hydrogène; et
- on ajoute une catalase immobilisée audit réactif au luminol pour décomposer le péroxide d'hydrogène dans le réactif au luminol.

20. Procédé selon la revendication 19, caractérisé en ce que ladite catalase immobilisée comprend une catalase immobilisée sur un support qui est formé par une réaction entre un support insoluble à l'eau et un réactif polyfonctionnel.

21. Appareil pour détecter la lumière produite par la réaction entre un réactif au luminol et du péroxide d'hydrogène dans un échantillon en présence d'un catalyseur pour mesurer la teneur du péroxide d'hydrogène dans l'échantillon, ledit appareil comprenant:
- un premier récipient dans lequel est stocké ledit réactif au luminol;
- un second récipient dans lequel est stocké un catalyseur;
- un boîtier pour définir une chambre creuse qui peut être rendue étanche sur le plan optique et dans laquelle est placée une cellule;
- une colonne garnie avec une catalase immobilisée, ladite catalase immobilisée étant active pour décomposer le péroxide d'hydrogène dudit réactif au luminol et dudit catalyseur;
- des premiers moyens pour injecter ledit réactif au luminol et ledit catalyseur à partir desdits premier et second récipients dans ladite cellule dans ladite chambre du boîtier à travers ladite colonne;
- des seconds moyens pour mesurer la quantité de lumière produite par la réaction entre ledit réactif au luminol et ledit péroxide d'hydrogène dudit échantillon en présence dudit catalyseur.

22. Appareil pour détecter la lumière produite par la réaction entre un réactif au luminol et du péroxide d'hydrogène dans un échantillon en présence d'un catalyseur, pour mesurer la teneur du péroxide d'hydrogène dans l'échantillon, ledit appareil comprenant:
- un premier récipient dans lequel sont stockés ledit réactif au luminol et une catalase immobilisée active pour décomposer le péroxide d'hydrogène dudit réactif au luminol;
- un second récipient dans lequel sont stockés ledit catalyseur et une catalase immobilisée active pour décomposer le péroxide d'hydrogène dudit catalyseur;
- un boîtier pour définir une chambre creuse qui peut être rendue étanche sur le plan optique et dans laquelle est placée une cellule;
- des premiers moyens pour injecter ledit réactif au luminol et ledit catalyseur depuis lesdits premier et second récipients dans ladite cellule dans ladite chambre du boîtier;
- des seconds moyens pour mesurer la quantité de lumière produite par la réaction entre ledit réactif au luminol et ledit péroxide d'hydrogène dudit échantillon en présence dudit catalyseur.
